# EUROPEAN PATENT SPECIFICATION

(11) **EP 0 148 486 B1**
(45) Date of publication and mention of the grant of the patent: **24.03.1993**
(21) Application number: 84116056.7
(22) Date of filing: 21.12.1984
(51) Int. Cl.: A61N 1/368

(54) **Improved rate adaptive pacemaker apparatus**
Sich an die Frequenz anpassendes Schrittmachergerät
Appareil d'entraînement se conformant à la fréquence

(30) Priority: 10.01.1984 US 569670
(43) Date of publication of application: 17.07.1985
(73) Proprietor: VITATRON MEDICAL B.V., NL-6950 AB Dieren (NL)
(72) Inventor: Wittkampf, Frederik H.M., NL-6771 LK Brummen (NL)
(74) Representative: Ebbinghaus, Dieter, Dipl.-Ing.

(56) References cited:
- EP-A- 0 077 845
- EP-A- 0 080 348
- EP-A- 0 096 464
- EP-A- 0 147 820
- DE-A- 3 243 094
- US-A- 3 921 642
- US-A- 4 228 803
- US-A- 4 390 022
- US-A- 4 401 119
- BIOMEDIZINISCHE TECHNIK, vol.20, no.6, 1975, Berlin, H.D.Funke: "Ein Herzschrittmacher mit belastungsabhängiger Frequenzregulation", pages 225-228

## Description

The invention relates to a demand pacer apparatus for pacing a patient, having a stimulus means for delivering stimulus pulses and sensing means for sensing natural heartbeats, first means for sensing a patient variable indicative of desired pacing rate, and rate means for modifying the rate of delivery of stimulus pulses by said stimulus means as a correlation function of said sensed patient variable. Such demand pacer apparatus is known from DE-A-32 43 094.

A physiologically adaptive cardiac pacemaker, wherein the desired pacing rate is determined as a function of sensed stimulus-T wave (Q-T) interval has been shown in US-A-4 228 803. This pacemaker, which is now being produced commercially, is referred to as the Tx rate adaptive pacemaker. The use of a microprocessor, or equivalent programmable circuitry in a pacemaker, such as shown in EP-A-0 077 845, makes the Tx pacer more readily feasible. Other improvements to this principle are disclosed in US-A-4 305 396.

The Tx rate adaptive pacer makes possible an improvement in determining the refractory interval for a ventricular (or atrial) pacer which has not been heretofore available. In conventional pacemakers, it is necessary to set the refractory interval safely after the expected time of occurrence of the T wave. However, this safety factor is at the expense of QRS sensing, since a longer refractory period results in a shorter sense period. In the Tx pacer, however, the occurrence of the T wave is sensed, and accordingly information is readily available for terminating the refractory period directly after the sensed T wave.

A normal premise of a rate adaptive demand pacemaker, Tx or otherwise, is that when the natural patient heart rate is being sensed and the pacemaker stimulus delivery is being inhibited, an optimum condition obtains. In other words, when the heart is able to operate without intervention of the pacemaker, it should do so. However, at the same time, there may be conditions when the pacemaker should overtake or overdrive the natural rate. For the Tx pacer, as long as pacing is inhibited, no Q-T data is acquired from which a pacing rate can be determined. Under such circumstances, it is desirable to get the Q-T rate indication and adjust the pacing rate, either to overtake the heart or to have the proper pacing rate established in the event of loss of the natural beat.

Another need of adaptive rate pacers generally is that of periodically checking and adjusting the correlation between a pacer indicated rate and the sensed physiological parameter, or data. For example, for the Tx pacer, a correlation or sensitivity function in terms of beats per minute (bpm) per Q-T interval (ms) must be programmed into the microprocessor; it is desirable to be able to check that correlation function and determine, at any point in the lifetime of the implanted pacemaker, whether the correlation function is appropriate, or whether it should be adjusted in view of data concerning the patient.

The prior art discloses demand pacers with rate means for modifying the rate of delivery of stimulus pulses in accordance with some correlation function of a sensed patient variable. Thus, DE-A 32 43 094 (US-A 4 467 807) shows a rate adaptive demand pacemaker, operative either as a single chamber or dual chamber pacemaker. In either case, pacing may intervene over the natural rate, or in the absence of a natural rate the pacing is controlled by and as a function of a sensed parameter (e.g., oxygen level, pH of the blood, respiration rate). However, such a system does not disclose any arrangement for checking or adjusting the correlation between the indicated pacing rate and the sensed parameter. What is needed is an improvement for determining when the correlation function may need to be changed, i.e., when it is not appropriate to indicate the patient's condition.

EP-A-0 077 845 discloses a dual chamber pacemaker which operates in an atrial synchronous mode when atrial signals are present; in the absence of atrial signals, ventricular pace pulses are delivered on a demand basis.

The intermediate document EP-A-0 147 820 discloses a dual chamber pacer operating in one or another of alternate rate adaptive modes. The pacemaker comprises means for automatically testing the appropriateness of the mode currently in use and for automatically selecting the appropriate rate control mode.

It is an object of this invention to provide a rate adaptive demand pacemaker which adjusts the correlation function between the rate of delivery of stimulus pulses and the sensed patient variable to the patient's requirements.

According to the present invention this object is solved - taking the demand pacer apparatus known from DE-A-3 243 094 as a basis - either by the characterizing features of claim 1 or of claim 9.

Preferred further developed developments and embodiments of the demand pacer apparatus of the present invention are described in claims 2 to 8 and 10 to 11.

The invention is further described with reference to the accompanying drawings in which:
Fig. 1 is a block diagram of a pacing system including an implantable pacemaker and external programming apparatus, as used in this invention.
Fig. 2a is a flow diagram of a Tx pacer with adjustable T wave sensing window and overdrive means.
Fig. 2b is a timing diagram illustrating the T wave detection window used in the Tx embodiment of this invention.
Fig. 3 is a flow diagram illustrating steps for checking the Tx correlation factor.
Fig. 4 is a flow diagram of a software program useful in this invention, incorporating means for changing the correlation factor between the pacing rate and sensed Q-T interval and for testing whether the patient natural rate is physiological or pathological.

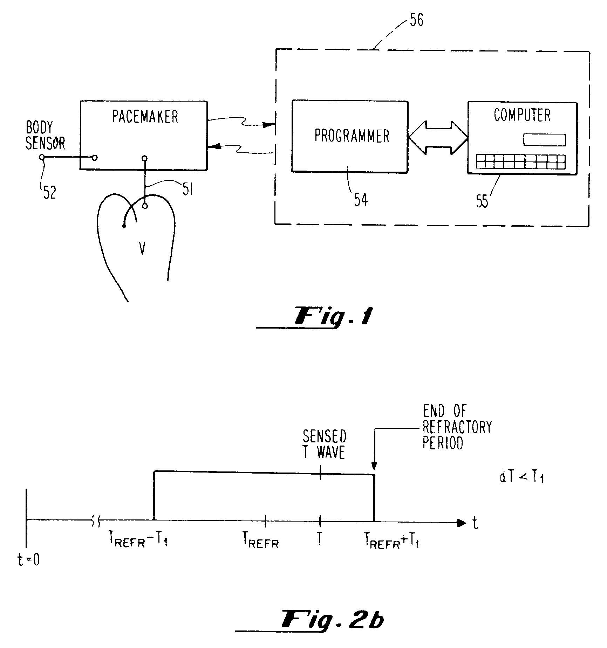

Reference is made to EP-A-0 077 845 which shows in detail an implantable pacemaker utilizing a microprocessor. As disclosed in the referenced application, the microprocessor pacemaker provides great flexibility, and different operating modes and routines can be easily incorporated by storing appropriate software in the pacemaker. As illustrated in the referenced application data may be transmitted between an external programmer to the pacemaker. Such external programming is now state of the art, and preferably part of the overall system into which this invention is incorporated, as illustrated in Fig. 1. The implantable pacemaker 50 is shown in two-way communication with external programming means 56. The external programming apparatus suitably comprises a programmer 54 which is positioned adjacent to the patient's heart, the programmer being in two-way communication with a computer 55, suitably an HP 85 computer. By this means, the operator can obtain operating data from the pacemaker and reprogram pacing variables in response thereto. Further, due to the memory capacity of the pacemaker, it is a relatively simple task to enable the pacemaker to operate in a number of different modes.

While the pacemaker of this invention is illustrated as being a simple single chamber, i.e., ventricular pacer, with a lead 51 connecting the pacemaker and the patient's ventricle, it is to be understood that the invention is applicable to atrial and dual chamber pacers. Also, there is illustrated a body sensor 52, which may be utilized to sense another body parameter such as respiration rate, the body parameter data being used for rate control purposes. More specifically, in the preferred embodiment of this invention, the pacemaker is a Tx type rate adaptive pacemaker such as disclosed in US-A-4 228 803. In such a pacemaker, the time interval from the delivered stimulus to the measured evoked T wave, referred to as the Q-T interval, is taken as an indicator of desired pacing rate, and means are provided for adjusting or controlling the pacing rate in terms of the sensed Q-T interval. As used herein, the phrase Q-T refers to either the time interval between a delivered stimulus and evoked T-wave when the pacemaker is pacing, or the time interval between the natural QRS and the T wave which follows, during pacemaker inhibited operation.

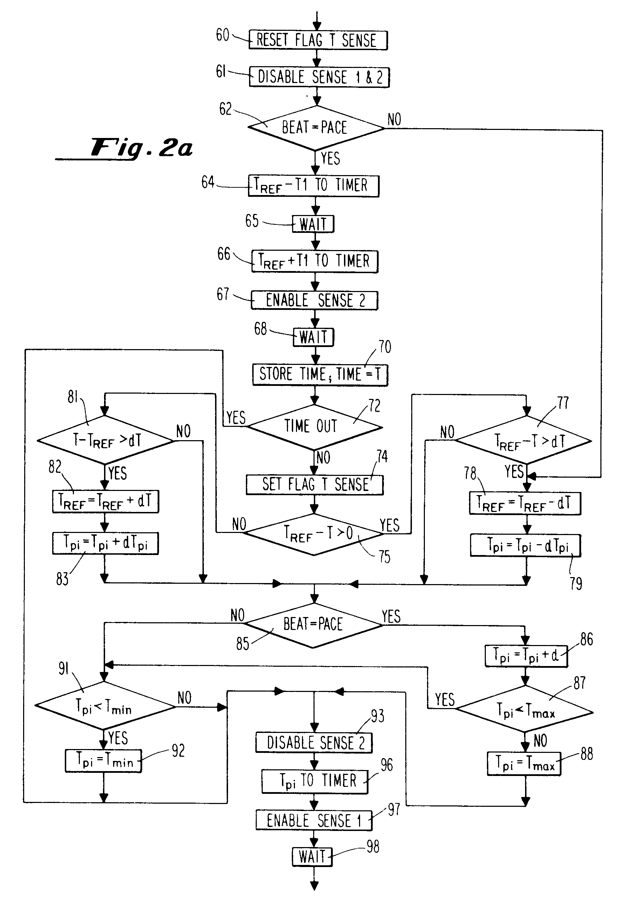

Referring to Fig. 2a, there is shown a flow diagram which illustrates an improved form of Tx operation, wherein there is incorporated an "overdrive" feature such that when inhibited operation is recognized the pacing interval is incrementally decreased until one or more stimulus signals are delivered, thereby enabling determination of the Tx-indicated rate. This procedure overcomes the problem that we have observed, namely that during inhibited operation the pacemaker is not receiving Q-T data, and as a result it lacks feedback as to whether the natural rate is physiologically good.

As illustrated, the flow diagram of Fig. 2a runs during the refractory period and starts just after pulse delivery or inhibition (not shown). As used in the flow diagram, the term "wait" means that the microprocessor is turned off and waits for start-up either by sensing an event or by timing out. Each block which indicates that a certain time is sent to the timer means that the pacemaker next looks for that time in the course of the pacing cycle.

At block 60, the pacemaker resets the T sense flag, and at block 61 disables the sense circuits 1 and 2, for sensing QRS and T waves respectively. At 62, the pacemaker determines whether the last interval was ended with a natural beat or a delivered stimulus pulse. If beat = pace, meaning that a stimulus was delivered, the pacemaker proceeds to set up the T wave window at blocks 64-68. Referring also to Fig. 2b, the pacemaker first puts the time T_{ref} - T₁ to the timer, which represents the start of the T wave window. At block 65, the pacemaker waits for that time to time out, and then sets the back edge of the window, T_{ref} + T1 to the timer at block 66, and enables the T wave sensing circuit at block 67. At block 68, the pacemaker waits, meaning that it is waiting through the time period of the window as illustrated in Fig. 2b. The wait is interrupted either by time out or a sensed T wave. At block 70 the pacemaker stores the time T, which represents the QT interval since the timing of the cycle starts at the time of ventricular stimulus or inhibition. At block 72, it is determined whether there has been a time out; if yes, the pacer branches to block 93.

If the determination at block 72 is that there has been no time out, meaning that the T wave was sensed, the T sense flag is set at block 74. It is next determined whether T_{ref} - T is greater than 0, i.e., whether the sensed T wave arrived in the first half or second half of the window. If the answer is yes, meaning that the T wave was sensed in the first half of the window, the program branches to block 77. It is then determined whether the difference is greater than a predetermined incremental value dT which may typically be 0.7 ms. If no, the program branches to block 85. If yes, the program goes to block 78 and subtracts the value of dT from T_{ref}. At block 79, the pacing interval Tₚᵢ is reduced by a value dTₚᵢ, suitably 6 ms. Thus, in response to the sensing of the QT interval having a decrease by more than an incremental amount, the T sense window is shifted minus dT and the pacing interval is reduced by the value Tₚᵢ. The values of dT and dTₚᵢ determine the slope setting, or the correlation factor between QT time and indicated change in pacing rate. A normal setting of a slope of 1 ppm/ms gives dT = 0.7 ms and dTₚᵢ = 6 ms, calculating the correlation value at a rate of 85 ppm. In the practice of this invention, it has been found that the slope or correlation factor should be maintained within the range of 0.5 to 2.0, in order to provide good rate tracking.

Returning to block 75, if the answer is no, meaning that the T wave was sensed after the middle of the window, then at block 81 it is determined whether the difference between T and T_{ref} is greater than dT. Again, if the answer is no, meaning that the change in QT interval was less than dT, the program branches to block 85. If the answer is yes, T_{ref} is incremented by + dT, and Tₚᵢ is incremented by + dTₚᵢ.

At block 85, it is determined whether the last cycle was ended with a delivered pacing stimulus. If yes, the program branches to block 86 and adds an increment d to the pacing interval. This provides a normal downward drift in pacing rate, when the pacemaker is delivering stimulus pulses. Note that if the sensed QT interval in subsequent cycles continues to indicate a higher rate, the timing change at block 78 will maintain the higher rate. However, in the absence of such QT information, the pacing rate will gradually drift downward to a predetermined minimum or base rate. At block 87, it is determined whether Tₚᵢ is less than the maximum pacing interval Tₘₐₓ; if not, Tₚᵢ is set equal to Tₘₐₓ at 88. If yes, the program branches to blocks 91 and 92, where the pacing rate is tested with respect to the minimum pacing interval Tₘᵢₙ. Note also that if, at block 85, it is determined that the last interval was terminated with a sensed QRS, the program likewise branches to block 91. Following this, at block 93 the sense 2 circuit for detecting the T wave is disabled. At block 96, the pacing interval Tₚᵢ is set to the timer, the sense 1 (QRS) circuit is enabled at 97, and at 98 the microprocessor waits until the next ventricular event initiates the new cycle. Note that if a T wave is sensed, the microprocessor handles certain steps and then the QRS sense is enabled at block 97, i.e., the refractory period is terminated upon sensing of the T wave. Alternately, upon detection of a T wave the pacer can still wait for time out of the refractory interval, if desired, before setting T ₚᵢ to the timer and enabling the QRS sense.

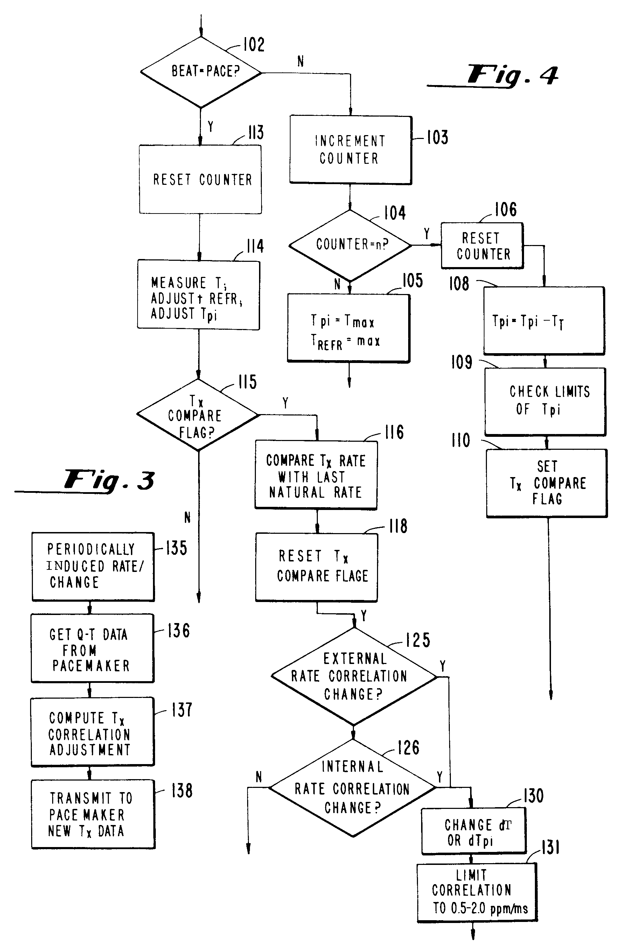

It is recognized that the correlation factor between Q-T and change in pacing rate may require adjustment following a certain length of pacing operation. Such adjustment may be made either through the external programming means, as illustrated by the flow diagram of Fig. 3, or may be made automatically by internal analysis. In Fig. 3, at block 135 the operator takes steps to periodically induce a rate change in the patient, by known means. At block 136, the pacemaker outputs QT data to the apparatus 56, where it is examined by the operator. At block 137, either the operator or the computer 55 computes the desired Tx correlation adjustment, and the Tx adjustment data is transmitted back to the pacemaker at block 138.

Referring now to Fig. 4, there is illustrated a flow diagram of a preferred embodiment of this invention, for automatically comparing a sensed patient natural rate with the indicated Tx rate. In this flow diagram, some of the details shown in Fig. 2a are omitted for brevity. At block 102, it is determined whether the last cycle ended with a pacing stimulus. If yes, a counter for counting successive natural sensed heartbeats is reset at block 113. At block 114, the steps for measuring the QT interval are carried out, and the pacing rate is adjusted accordingly. At block 115, the microprocessor determines whether there is a Tx compare flag, i.e., whether the program is to compare the Tx indicated rate with the last natural rate. If not, the routine carries out the limit checks as shown in Fig. 2a, and exits. If, at block 115, it is found that the Tx compare flag is set, then at block 116 the microprocessor compares the Tx indicated rate with the last natural rate. This is a programmed logical step and may, for example, be a comparison of the difference of the Tx rate and the natural rate with a predetermined limit. In other words, if the natural rate is found to differ from the Tx indicated rate by more than the predetermined limit, it is deemed that the correlation, i.e., sensitivity function, is not acceptable. In making the comparison, either single cycle values of natural rate and Tx-indicated rate can be compared, or mean values taken over plural cycles can be compared. Following this, the Tx Compare Flag is reset at block 118.

The pacemaker next proceeds to blocks 125 and 126, to determine whether any correlation change is required. At block 125, the pacemaker determines whether any adjustment of the correlation has been signaled from the external programmer, such as illustrated at Fig. 3. If yes, the programmed change in either dT or dTₚᵢ is carried out at block 130. If no external rate correlation change is flagged, then the pacemaker proceeds to block 126 and determines whether internally generated data indicates a rate correlation change. This data can come from sensor 52, or it can be produced by the logical analysis made at block 116. If a rate correlation change is indicated, it is made at block 130. At block 131 a check is made to limit the correlation to the predetermined range, preferably 0.5 - 2.0 ppm/ms.

Returning to block 102, if it is determined that the last interval ended in a sensed QRS, the counter 103 is incremented. At 104, it is determined whether the counter has reached a predetermined number n, representative of an arbitrary number of successive sensed natural heartbeats. If no, the pacing interval and refractory interval are set at 105, and the routine exits. If the counter has reached n, the program branches to block 106, where the counter is reset. At block 108, the pacing interval is adjusted by subtracting the increment T_{T}. T_{T} may suitably be 10 to 20 ms, sufficient to cause the pacemaker to insert an early stimulus. Thus, depending on the setting of the counter at 104, every n cycles of inhibited operation an early stimulus is delivered so that a Tx-indicated rate determination can be made. At block 109, the limits of Tₚᵢ are checked, and at block 110 the Tx compare flag is set. The routine then exits, following which a stimulus is delivered, enabling measurement of the QT interval during the next cycle at block 114.

The embodiment of Figure 4 illustrates a single overdrive stimulus which is specially timed to be delivered just before the next expected spontaneous beat, so as to obtain a Q-T value without the pacer really intervening in the patient's natural rhythm. However, it is to be understood that, if desired, the routine can be changed to continuously decrease Tₚᵢ over a plurality of cycles until a stimulus is delivered, and to deliver a plurality of stimuli. Thus, for example, following block 106 an "Overdrive" flag may be set which causes the program to branch from block 102 (assuming No response there) to block 108 each pacer cycle until intervention is achieved.

## Claims

1. Rate adaptive demand pacer apparatus (50) for pacing a patient, having sensing means for sensing natural heartbeats and stimulus means for delivering stimulus pulses at a controllable rate in the absence of sensed natural heartbeats,
first means for sensing a first patient variable indicative of desired pacing rate (70 or 114), and
rate means for controlling the rate of delivery of stimulus pulses by said stimulus means as a correlation function of said first sensed patient variable (114), characterized by:
second means for sensing a second patient variable indicative of desired pacing rate (116, 126), and correlation adjusting means for adjusting said correlation function when said second patient variable indicates a correlation change (130), whereby the correlation function is adjusted to more accurately represent desired pacing rate in terms of patient conditions.

2. The pacer apparatus as described in claim 1, wherein said first means comprises QT means (70) for sensing the QT interval of a cycle of said patient.

3. The demand pacer apparatus as described in claim 1, wherein said second means comprises means (52) for sensing the respiratory rate of the patient.

4. The apparatus as described in claim 2, wherein said QT interval is sensed following delivery of a stimulus pulse, and said rate means controls the pacing interval of said stimulus means as a function of the sensed QT interval following each delivered stimulus (102, 114).

5. The apparatus as described in claim 4, wherein said rate means comprises means for decreasing said pacing interval at a predetermined rate following sensing of a natural heartbeat (108), whereby said pacer rate rises so that a stimulus pulse is delivered after one or more naturally occurring heartbeats and said QT interval is again measured.

6. The apparatus as described in claim 1, comprising limit means (131) for limiting said correlation function within a predetermined limit.

7. The apparatus as described in claim 1, wherein said second means comprises means for accumulating patient data over a plurality of cycles (126).

8. The apparatus as described in claim 1, wherein said second means further comprises external means (125, Fig. 3) for processing sensed patient data.

9. Rate adaptive demand pacer apparatus (50) for pacing a patient, having ventricular sensing means for sensing natural ventricular heartbeats and stimulus means for delivering ventricular stimulus pulses at a controllable rate in the absence of sensed natural ventricular heartbeats,
means for sensing a patient variable indicative of desired pacing rate (70 or 114), and
rate means for controlling the rate of delivery of stimulus pulses by said stimulus means as a correlation function of said sensed patient variable (114), characterized by:
means for sensing the patient's natural atrial rate (116), and correlation adjusting means for adjusting said correlation function when said natural atrial rate indicates a correlation change (26, 30), whereby the correlation function is adjusted to more accurately represent desired pacing rate in terms of patient conditions.

10. The apparatus as described in claim 1, wherein said second means has a body sensor (52) positioned outside of the patient's heart for sensing said second patient variable.

11. The apparatus as described in claim 1, having a pacemaker (50), a lead (51) connected between said pacemaker and at least one chamber of the patient's heart, and wherein said second means has a sensor (52) separate from said lead.

## Patentansprüche

1. Bedarfs-Schrittmachergerät (50) mit anpaßbarer Frequenz, zur Versorgung eines Patienten mit Schrittmacherimpulsen, mit einer Fühleinrichtung zum Erfassen natürlicher Herzschläge und einer Reizeinrichtung zum Abgeben von Reizimpulsen mit steuerbarer Frequenz bei Fehlen erfaßter natürlicher Herzschläge,
einer ersten Einrichtung (70 oder 114) zum Erfassen einer ersten Variablen des Patienten, die die Soll-Schrittfrequenz anzeigt, und
einer Frequenzeinrichtung (114) zum Steuern der Frequenz der Abgabe von Reizimpulsen durch die Reizeinrichtung als Korrelationsfunktion der ersten erfaßten Variablen des Patienten, **gekennzeichnet** durch:
eine zweite Einrichtung (116, 126) zum Erfassen einer zweiten Variablen des Patienten, die die Soll-Schrittfrequenz anzeigt, und eine Korrelations-Einstelleinrichtung (130) zum Einstellen der Korrelationsfunktion, wenn die zweite Variable des Patienten eine Korrelationsänderung anzeigt, wobei die Korrelationsfunktion so eingestellt wird, daß sie die Soll-Schrittfrequenz bezogen auf den Zustand des Patienten genauer darstellt.

2. Schrittmacher nach Anspruch 1, wobei die erste Einrichtung eine QT-Einrichtung (70) zum Erfassen des QT-Intervalls eines Zyklus des Patienten aufweist.

3. Schrittmachergerät nach Anspruch 1, wobei die zweite Einrichtung eine Einrichtung (52) zum Erfassen der Atemfrequenz des Patienten aufweist.

4. Schrittmachergerät nach Anspruch 2, wobei das QT-Intervall folgend auf die Abgabe eines Reizimpulses erfaßt wird, und wobei die Frequenzeinrichtung (102, 114) das Schrittmacherintervall der Reizeinrichtung als Funktion des erfaßten QT-Intervalls nach jedem abgegebenen Reizimpuls steuert.

5. Schrittmachergerät nach Anspruch 4, wobei die Frequenzeinrichtung eine Einrichtung (108) zum Vermindern des Schrittmacherintervalls auf eine vorbestimmte Frequenz aufweist, nachdem ein natürlicher Herzschlag erfaßt wurde, wobei die Schrittfrequenz so ansteigt, daß ein Reizimpuls nach einem oder mehreren natürlich auftretenden Herzschlägen abgegeben wird und das QT-Intervall wieder gemessen wird.

6. Schrittmachergerät nach Anspruch 1, mit einer Begrenzungseinrichtung (131) zum Begrenzen der Korrelationsfunktion innerhalb vorbestimmter Grenzen.

7. Schrittmachergerät nach Anspruch 1, wobei die zweite Einrichtung eine Einrichtung (126) zum Sammeln von Daten des Patienten über mehrere Zyklen aufweist.

8. Schrittmachergerät nach Anspruch 1, wobei die zweite Einrichtung ferner externe Einrichtungen (125, Figur 3) zum Verarbeiten erfaßter Daten des Patienten aufweist.

9. Bedarfs-Schrittmachergerät (50) mit anpaßbarer Frequenz zur Versorgung eines Patienten mit Schrittmacherimpulsen, mit einer ventrikularen Fühleinrichtung zum Erfassen natürlicher ventrikularer Herzschläge und einer Reizeinrichtung zur Abgabe ventrikularer Reizimpulse mit steuerbarer Frequenz bei Fehlen erfaßter natürlicher ventrikularer Herzschläge,
einer Einrichtung (70 oder 114) zum Erfassen einer Variablen des Patienten, die die Soll-Schrittfrequenz anzeigt, und
einer Frequenzeinrichtung (114) zum Steuern der Frequenz der Abgabe von Reizimpulsen durch die Reizeinrichtung als Korrelationsfunktion der erfaßten Variablen des Patienten, **gekennzeichnet** durch
eine Einrichtung (116) zum Erfassen der natürlichen atriellen Frequenz des Patienten, und durch eine Korrelationseinstelleinrichtung (26, 30) zum Einstellen der Korrelationsfunktion, wenn die natürliche atrielle Frequenz eine Korrelationsänderung anzeigt, wobei die Korrelationsfunktion so eingestellt wird, daß sie die Soll-Schrittfrequenz bezogen auf den Zustand des Patienten genauer darstellt.

10. Schrittmachergerät nach Anspruch 1, wobei die zweite Einrichtung einen Körperfühler (52) zum Erfassen der zweiten Variablen des Patienten aufweist, der außerhalb des Herzens des Patienten angeordnet ist.

11. Schrittmachergerät nach Anspruch 1, mit einem Schrittmacher (50), einer Leitung (51) zwischen dem Schrittmacher und wenigstens einer Kammer des Herzens des Patienten, wobei die zweite Einrichtung einen vom Leiter getrennten Sensor (52) aufweist.

## Revendications

1. Appareil stimulateur cardiaque (50) à la demande, adaptatif en rythme, destiné à stimuler un patient, comprenant des moyens détecteurs pour détecter des battements cardiaques naturels et un moyen de stimulus pour envoyer, en l'absence de battements cardiaques naturels détectés, des impulsions de stimulus à un rythme réglable,
un premier moyen de détection d'une première variable du patient indicative d'un rythme souhaité de stimulation (70 ou 114), et
un moyen de rythme prévu pour régler le rythme d'envoi d'impulsions de stimulus par ledit moyen de stimulus en tant que fonction de corrélation de ladite première variable détectée (114) du patient, caractérisé par
un deuxième moyen de détection d'une deuxième variable du patient indicative d'un rythme souhaité de stimulation (116, 126), et un moyen d'ajustement de corrélation pour ajuster ladite fonction de corrélation lorsque ladite deuxième variable du patient indique une variation de corrélation (130), grâce à quoi la fonction de corrélation est ajustée afin de représenter plus précisément un rythme de stimulation souhaité en termes de conditions du patient.

2. Appareil stimulateur comme décrit dans la revendication 1, dans lequel ledit premier moyen comprend un moyen QT (70), pour détecter l'intervalle QT d'un cycle dudit patient.

3. Appareil stimulateur cardiaque à la demande comme décrit dans la revendication 1, dans lequel ledit deuxième moyen comprend un moyen (52) pour détecter le rythme respiratoire du patient.

4. Appareil comme décrit dans la revendication 2, dans lequel ledit intervalle QT est détecté à la suite de l'envoi d'une impulsion de stimulus, et ledit moyen de rythme règle l'intervalle de stimulation dudit moyen de stimulus en fonction de l'intervalle détecté QT à la suite de chaque stimulus envoyé (102, 114).

5. Appareil comme décrit dans la revendication 4, dans lequel ledit moyen de rythme comprend un moyen prévu pour diminuer ledit intervalle de stimulation à un rythme prédéterminé à la suite de la détection d'un battement cardiaque naturel (108), grâce à quoi ledit rythme de stimulateur augmente d'une manière telle qu'une impulsion de stimulus est envoyée après un ou plusieurs battements cardiaques se produisant de façon naturelle et ledit intervalle QT est mesuré à nouveau.

6. Appareil comme décrit dans la revendication 1, comprenant un moyen limiteur (131) pour limiter ladite fonction de corrélation à l'intérieur d'une limite prédéterminée.

7. Appareil comme décrit dans la revendication 1, dans lequel ledit deuxième moyen comprend un moyen d'accumulation des données du patient sur une pluralité de cycles (126).

8. Appareil comme décrit dans la revendication 1, dans lequel ledit deuxième moyen comprend en outre un moyen externe (125, Fig. 3) de traitement de données détectées du patient.

9. Appareil stimulateur cardiaque à la demande (50) adaptatif en rythme destiné à stimuler un patient, comprenant un moyen de détection ventriculaire, prévu pour détecter des battements cardiaques ventriculaires naturels, et un moyen de stimulus prévu pour envoyer des impulsions de stimulus ventriculaire à un rythme réglable en l'absence de battements cardiaques ventriculaires naturels détectés,
un moyen de détection d'une variable du patient indicative d'un rythme souhaité de stimulation (70 ou 114), et
un moyen de rythme prévu pour régler le rythme d'envoi d'impulsions de stimulus par ledit moyen de stimulus en tant que fonction de corrélation de ladite variable (114) détectée du patient, caractérisé par
un moyen de détection du rythme naturel (116) auriculaire du patient et un moyen d'ajustement de corrélation pour ajuster ladite fonction de corrélation lorsque ledit rythme naturel auriculaire indique une variation de corrélation (26, 30), grâce à quoi la fonction de corrélation est ajustée pour représenter plus précisément un rythme souhaité de stimulation en termes de conditions de patient.

10. Appareil comme décrit dans la revendication 1, dans lequel ledit deuxième moyen comprend un détecteur corporel (52) positionné à l'extérieur du coeur du patient pour détecter ladite deuxième variable de patient.

11. Appareil comme décrit dans la revendication 1, comprenant un stimulateur cardiaque (50), un conducteur (51) relié entre ledit stimulateur cardiaque et au moins une cavité du coeur du patient, et dans lequel ledit deuxième moyen comprend un détecteur (52) séparé dudit conducteur.
